# EUROPEAN PATENT SPECIFICATION

(11) **EP 3 315 103 B1**
(45) Date of publication and mention of the grant of the patent: **17.03.2021**
(21) Application number: 17199051.8
(22) Date of filing: 27.10.2017
(51) Int. Cl.: A61F 5/02

(54) **GARMENT INCLUDING A T-SHIRT AND A SYSTEM TO CORRECT THE POSTURE OF A USER**
KLEIDUNGSSTÜCK MIT EINEM T-SHIRT UND SYSTEM ZUR KORREKTUR DER HALTUNG EINES TRÄGERS
VÊTEMENT COMPRENANT UN T-SHIRT ET UN SYSTÈME POUR CORRIGER LA POSTURE D'UN UTILISATEUR

(30) Priority: 27.10.2016 IT 201600108678
(43) Date of publication of application: 02.05.2018
(73) Proprietor: Dual Sanitaly S.p.A., 10121 Torino (IT)
(72) Inventor: RANGONI, Eugenio, 10121 Torino (IT); RANGONI, Francesco, 10121 Torino (IT); CONTI, Marco, 10121 Torino (IT); GAROFALO, Raffaele, 10121 Torino (IT)
(74) Representative: Fiussello, Francesco

(56) References cited:
- EP-A1- 1 398 010
- WO-A1-2009/052031
- WO-A1-2013/138468
- US-A1- 2005 197 607
- US-A1- 2014 018 715

## Description

### TECHNICAL FIELD

The invention relates to a garment comprising a t-shirt and a system to correct the posture of a user.

### PRIOR ART

The importance of a good posture is known among health professionals. "Posture" refers to the alignment of the body and, more specifically, to the relative static and dynamic positioning of the body and of the limbs.

With a good posture, the spine has no lateral curvature and the legs have no angles in the knees and ankles. Furthermore, when viewed from the side, the spine forms a slightly S-shaped curve, with the two curves of the S-shape evenly arranged on opposite sides of an imaginary line extending downward from the head through the centre of the body.

This alignment provides an even distribution of the body weight over the spine and a relatively even distribution of the pressure on the inter-vertebral discs of the spine.

On the other hand, the effects of a poor posture are well documented. For example, in the long run, a hunched position of the upper part of the body results in a contraction of any possible movement, in particular of the pectoral muscles in the thorax.

Other effects of a poor posture include discomforts, such as headaches and pain in the shoulders, arms and hands. Furthermore, a poor posture can cause pain in the jaw due to a forward position of the head as well as a decreased lung capacity due to the volume decrease of the thoracic cavity.

One of the most common consequences of a poor posture is the onset of back pain that can increase with advancing age.

However, a posture can be corrected to distribute loads more evenly.

It should be noted that maintaining a good posture is important, both when sitting and standing and during various activities, such as walking, running, etc.

It is also known that an increasingly sedentary lifestyle, in which many hours are spent sitting in front of a computer, driving a car, or watching television, has a negative effect on posture. Such static activities may cause a forward protrusion of the head and neck, a rounding of the thorax and of the lumbar spine and a stretching of the ligaments of the spine.

A poor posture during dynamic activities such as running or lifting results in inefficient body movements, with increased stress on muscles and ligaments.

Maintaining a correct posture during static and dynamic activities can avoid an excessive stress on muscles and ligaments. Furthermore, maintaining a correct posture can train some muscles through muscle memory so that a correct posture will eventually become a habit. However, it is sometimes difficult to be continuously aware of one's correct posture during mentally stimulating activities, such as working at a computer or during physically demanding activities, such as during sports practice. Physiotherapists can use a postural therapy to improve the patient's posture. Such postural therapy may include techniques such as shoulder taping and/or breathing exercises. Conventional chiropractic techniques can also use body manipulation and various treatments to help keep the patient's spine in alignment.

However, the patient must go to the physiotherapist or chiropractor in order to receive the necessary postural therapy.

Therefore, there is a need for a device that is specifically suitable for a continuous training and development of some body muscles so that the patient's posture can be improved.

Furthermore, there is a need for a device that allows this type of muscle training even while sitting.

Furthermore, there is a need for a device that allows this muscle training during various activities such as walking, running and other daily activities.

Furthermore, there is a need in the art for a device to improve the posture of a patient that can be progressively adjusted, depending on changes, or can improve the patient's posture over time.

Finally, there is a need in the art of a device to improve the posture of a patient that can be adjusted according to various physiological parameters of the patients, such as age, body size, muscle development and pathophysiological status.

US2014 / 018715 discloses a spinal orthosis having an elongate and substantially rigid spinal frame having upper, middle and lower portions, and a lumbar assembly connected to the lower portion of the spinal frame. A strap assembly secures to an upper portion of the spinal frame and is adjustably secured to the lumbar assembly. An elastic strap has a first end secured to the spinal frame and a second end adjustably securing to the lumbar assembly.

US 2005/197607 discloses a device for improving the posture of a patient comprising a garment and a pair of tension straps.

### SCOPE OF THE INVENTION

The object of the present invention is to provide a garment comprising a t-shirt and a system to correct the posture of a user, which can solve the aforesaid problems and which can be easily produced and marketed.

According to the present invention, the aforementioned problems are solved by a garment comprising a t-shirt and a system to correct the posture of a user according to claim 1.

### BRIEF DESCRIPTION OF THE DRAWINGS

- Figure 1 is a rear view of a garment comprising a t-shirt and a first embodiment of a system to correct the posture of a user;
- Figure 2 is a front view of a garment comprising a t-shirt and a first embodiment of a system to correct the posture of a user;

- Figure 3 is a side view of a garment comprising a t-shirt and a first embodiment of a system to correct the posture of a user;
- Figure 4 is a rear view of a garment comprising a t-shirt and a second embodiment of a system to correct the posture of a user;
- Figure 5 is a front view of a garment comprising a t-shirt and a second embodiment of a system to correct the posture of a user; and
- Figure 6 is a side view of a garment comprising a t-shirt and a second embodiment of a system to correct the posture of a user.

### DETAILED DESCRIPTION OF THE INVENTION

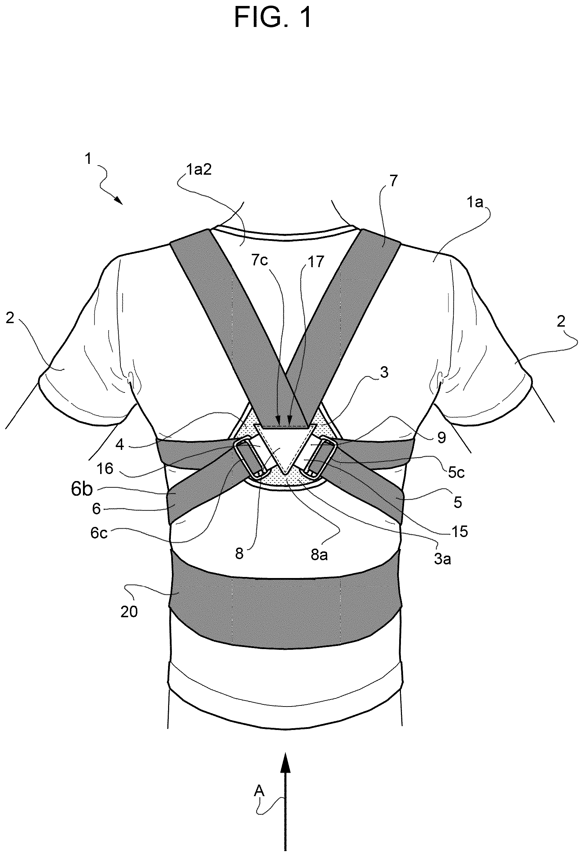

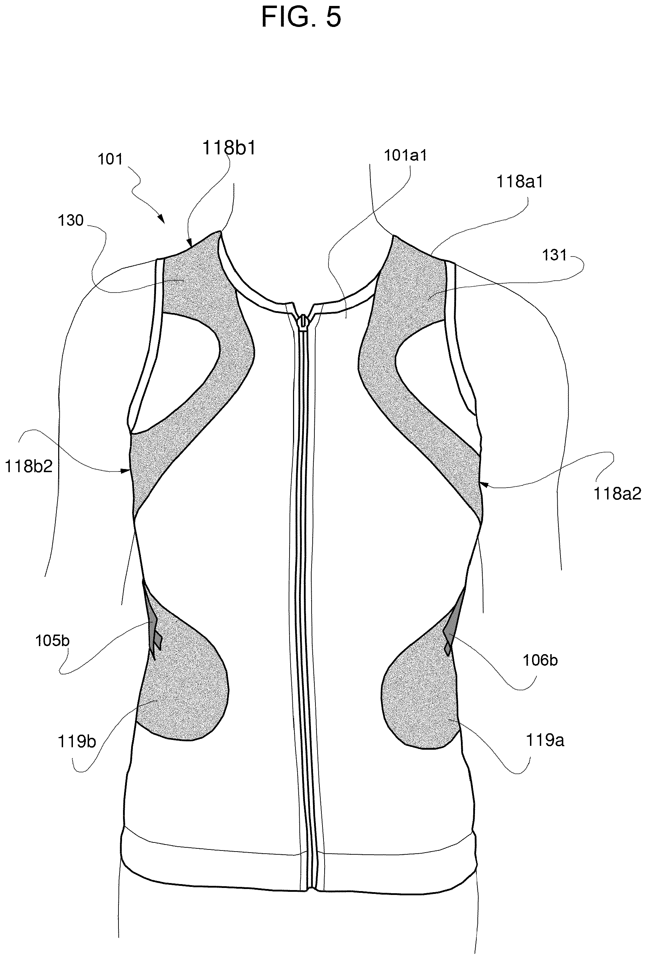

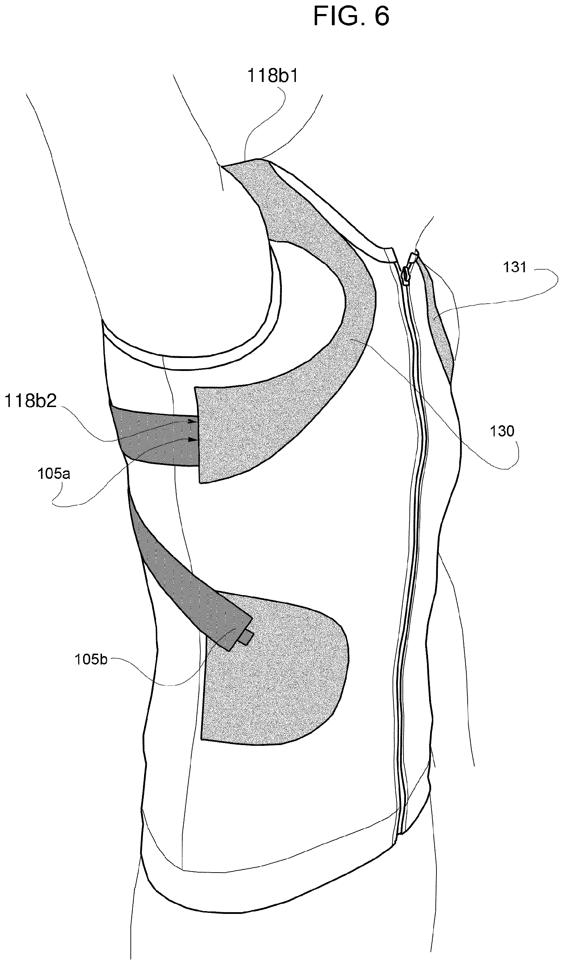

In the following, reference will be made in particular to the embodiments of the invention, which are shown in Figures 1 to 6, without thereby intending to limit the invention to such embodiments.

In the following, any reference to a body position or direction will be made with respect to a body in an upright position, as shown in Figure 1.

With reference in particular to Figures 1-3, in a first embodiment the reference number 1 indicates as a whole a garment comprising a t-shirt 1a, preferably a t-shirt 1a with two short sleeves 2. Alternatively, the sleeves may also be long or t-shirt can also mean a vest or a shirt without sleeves.

Back and torso of the shirt mean the respective relative t-shirt portions that, when the t-shirt is worn, are on the user's back and torso.

The t-shirt 1a can be made of any material, such as cotton or other material that can be used for making clothes.

The shirt includes a torso 1a1 and a back 1a2.

Preferably, the t-shirt 1a is made of an elastic and/or transpiring material, more preferably the t-shirt is made of a fabric with an elastic behaviour with two different modules in the two main directions, even more preferably also washable and quick-drying.

Preferably, the t-shirt 1a comprises one or more fabrics having different characteristics.

The garment 1 also includes a system 3 to correct the posture of a user.

Preferably, the system 3 to correct the posture of a user comprises a panel 4 and a plurality of elastic strips 5, 6 and 7.

Elastic strips means strips of material provided with intrinsic elasticity and which therefore can be elongated or stretched tending to return to the original length.

The panel 4 is preferably arranged approximately on the back, preferably in the middle of the back, when the t-shirt 2 is worn, more preferably at the height of the sternum.

The panel 4 preferably comprises a fabric and preferably comprises a fastening system, more preferably releasable, for a coupling system 8.

The coupling system 8 is preferably used for fastening the strips 5, 6 and 7.

Preferably, the coupling system 8 is releasably fastened on the panel 4, but can also be fastened in a non-releasable manner.

Preferably, the coupling system is fastened on the panel 4 by means of fastening means 3a. Preferably, the fastening means comprise a hook and loop or Velcro® system.

As already known, the hook and loop or Velcro® system consists of a removable fastening system comprising a first hooked strip, preferably made of nylon and comprising a plurality of small hooks, and a second strip of hairy material, also commonly referred to as velour or looped material to which the hooks are attached, thus ensuring the system fastening.

Preferably, the hook and loop or Velcro® system 8 consists of a first strip of hairy material fastened on and forming an integral part of the panel 4 and a second hooked strip (not shown) arranged below the coupling system 8.

The coupling system 8 is also preferably provided with a plurality of sliding or retaining means indicated in Figure 1 as a whole with the number 9.

Sliding or retaining means 9 are means that allow the elastic strips 5, 6, 7 to slide.

The retaining means 9 may be made of through buckles, as in Figure 1, of a simple notch in the coupling system or of any other means that allow inserting an elastic strip and make it slide.

In the embodiment shown in Figures 1 to 3, the retaining means 9 are preferably made of through buckles 15, 16 since the buckles easily allow sliding and then adjusting the length of the elastic strips, and/or of a notch 17.

Preferably, the system 1 to protect the posture comprises three elastic strips 5, 6 and 7 connected to the coupling system 8.

Only the simultaneous presence of the three elastic strips 5, 6 and 7 allows keeping the user in a correct posture both during dynamic and static activities.

The elastic strips 5, 6 and 7 comprise respective ends 5a, 5b, 6a, 6b, 7a, 7b and central portions 5c, 6c, 7c.

Ends mean not only the actual end, but also the whole end portion of the strip suitable to be fastened to a respective fastening portion.

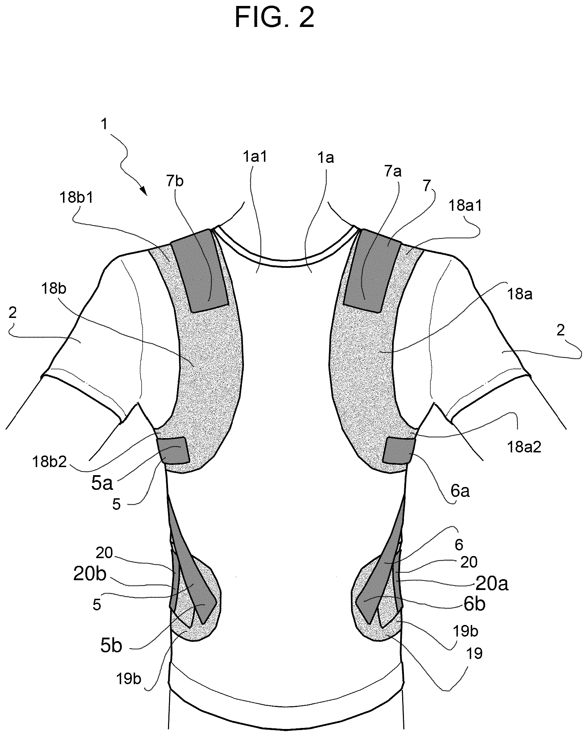

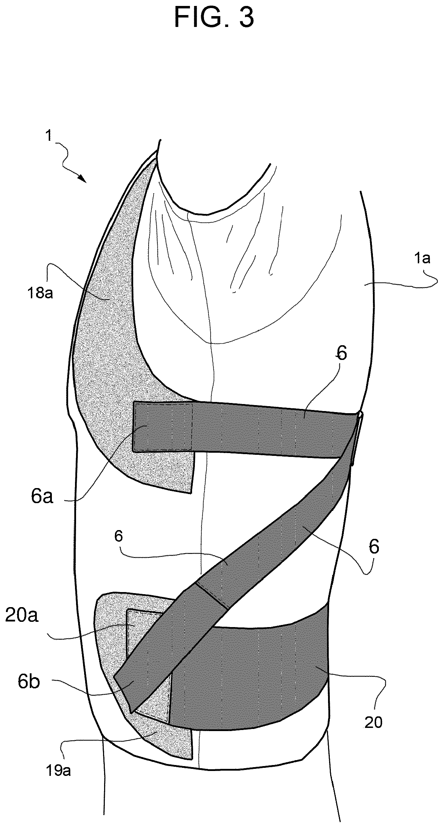

More in detail and as shown in detail in Figures 2 and 3, the T-shirt 1a comprises on the torso 1a1:
- a first fastening portion 18a1 arranged on the upper portion of the torso between the left shoulder and the neck,
- a second fastening portion 18b1 arranged on the upper portion of the torso between the right shoulder and the neck,
- a third fastening portion 18a2 arranged at the height of the left hypochondrium,
- a fourth fastening portion 18b2 arranged at the height of the right hypochondrium,
- a fifth fastening portion 19a arranged on the left side,
- a sixth fastening portion 19b arranged on the right side.

A first end 5a of the first elastic strip 5 is fastened to the fourth fastening portion 18b2. The central portion 5c of the first strip engages the retaining means 9 of the coupling system 8 and in particular engages the buckle 15. The second end 5b of the first strip 5 is fastened to the sixth fastening portion 19b.

A first end 6a of the second elastic strip 6 is fastened to the third fastening portion 18a2. The central portion 6c of the second strip engages the retaining means 9 of the coupling system 8 and in particular engages a buckle 16. The second end 6b is fastened to the fifth fastening portion 19a.

A first end 7a of the third elastic strip 7 is fastened to the first fastening portion 18a1. The central portion 7c of the third strip engages the retaining means 9 of the coupling system 8 and in particular engages the notch 17.

The second end 7b is fastened to the second fastening portion 18b1.

The tension of the elastic strips by positioning the elastic strips on the respective fastening portions 18a1, 18a2, 18b1, 18b2, 19a, 19b is adjusted to allow correcting the user's posture.

In detail, when the user wears the shirt, she/he fastens the elastic strips 5, 6 and 7 on the respective fastening portions and, by adjusting the tension, adjusts the posture correction.

In fact, the more stretched the elastic strips, the greater the posture correction effect.

Preferably, the elastic strips 5, 6, 7 are releasably fastened to the respective fastening portions.

More preferably, by means of a hook and loop system, even more preferably with a hook and loop system known as Velcro®.

Preferably, the fastening portions 18a1, 18a2, 18b1, 18b2, 19a, 19b are made of strips of hairy material, while the strips 5, 6 and 7 have hooked strips not shown at their respective ends.

The posture correction system 3 allows an effective correction only in combination with the t-shirt.

The fastening portions 18a1, 18a2, 18b1, 18b2, 19a, 19b are fastened to the t-shirt 1a preferably by seaming or heat-sealing.

Advantageously, the first fastening portion 18a1 and the second fastening portion 18a2 can form a single wide fastening area 18a, which extends from the left shoulder to the left hypochondrium.

Advantageously, the third fastening portion 18b1 and the fourth fastening portion 18b2 can form a single, wide fastening area 18b, which extends from the right shoulder to the right hypochondrium.

In this way, preferably, the fastening portions 18a and 18b extend along a torso strip comprised between the shoulders and the hypochondrium at the user's side.

Advantageously, the fifth fastening portion 19a and the sixth fastening portion 19b can form a single fastening portion 19 along the whole lumbar back portion.

The elastic strips 5, 6 and 7 have a suitable width to have an effective action in correcting the posture and which is preferably comprised between 3 and 10 cm.

The width of the elastic strips 5, 6 and 7 can be variable along their length.

The elastic strips 5, 6 and 7 have a suitable length to be fastened in different positions on the fastening portions 18a, 18b and 19 so as to ensure an adjustment of the position and therefore a greater or lesser effect of posture correction and so that the garment is tailored on the user's size.

Therefore, the strips 5, 6 and 7 preferably have a length comprised between 25 cm and 60 cm.

Optionally and preferably, it is possible that the garment 1 comprises further elastic strips.

Advantageously, the garment 1 could optionally comprise a fourth elastic strip 20 arranged around the lumbar strip and in particular connecting the portions 19a and 19b.

Preferably, the elastic strips 5 and 6 are fastened to the respective fastening portions at more convenient angles to ensure a maximum posture correction.

More preferably, the elastic strip 7 is fastened at an angle comprised between 0° and 30° with respect to the vertical axis A, more preferably comprised between 5° and 20°.

More preferably, the elastic strip 5 has the upper end 5b fastened at an angle comprised between 75° and 105° with respect to the vertical axis A.

More preferably, the elastic strip 5 has the lower end 5a fastened at an angle comprised between 120° and 150° with respect to the vertical axis A.

More preferably, the elastic strip 6 has the upper end 6b fastened at an angle comprised between 75° and 105° with respect to the vertical axis A.

More preferably, the elastic strip 6 has the lower end 6a fastened at an angle comprised between 120° and 150° with respect to the vertical axis A.

More preferably, the ends 6a, 5a of the elastic strips 5, 6 are fastened on the respective ends 20a, 20b of the elastic strip 20.

Optionally, the t-shirt 1a can be provided with a front hinge, extending along an axis B orthogonal to the axis A and preferably in a central position with respect to the user's chest and allowing its being worn by users who have a limited ability to move.

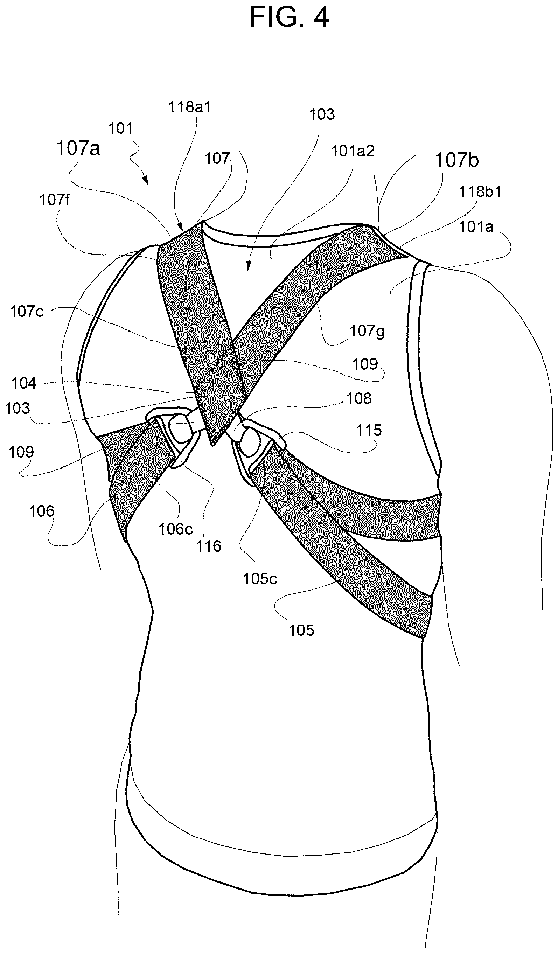

According to a second embodiment and with reference in particular to Figures 4 to 6, 101 indicates as a whole a garment comprising a sleeveless vest 101a. Alternatively, the sleeves may also be long or short.

The back and torso of the shirt indicate the respective t-shirt portions that, when the t-shirt is worn, are on the user's back and torso.

The vest 101a can be made of any material such as cotton or any other material that can be used for making clothes.

The vest includes a torso 101a1 and a back 101a2.

Preferably, the vest 101a is made of an elastic and/or transpiring material, more preferably the t-shirt is made of a fabric with an elastic behaviour with two different modules in the two main directions, even more preferably also washable and quick-drying.

Preferably, the vest 101a comprises one or more fabrics having different characteristics.

The garment 101 further comprises a system 103 to correct the posture of a user.

Preferably, the system 103 to correct the posture of a user comprises a panel 104 and a plurality of elastic strips 105, 106 and 107.

Elastic strips mean strips of material provided with intrinsic elasticity and which therefore can be elongated or stretched tending to return to the original length.

The panel 104 is preferably arranged approximately on the back, preferably in the centre of the back, when the t-shirt 102 is worn, more preferably at the height of the sternum.

The panel 104 preferably comprises a fastening system, more preferably releasable, for a coupling system 108.

The coupling system 108 is preferably used for fastening the strips 105, 106 and 107.

Preferably, the coupling system 108 is fastened in a non-releasable manner on the panel 104, but it can also be releasably fastened. Moreover, the panel 104 may also not be there and the coupling system 108 may be directly fastened on the t-shirt 102.

The coupling system 108 is also preferably provided with a plurality of sliding or retaining means indicated as a whole in Figure 4 with the number 109.

Sliding or retaining means 109 are means that allow the elastic strips 105, 106, 107 to slide or to be fastened.

The retaining means 109 can be made of through buckles, as in Figure 4 for the elastic strips 105 and 106, or of seams as for the elastic strip 107. Alternatively, they can also be made of a simple notch in the coupling system or of any other means that allow inserting an elastic strip and fastening it or make it slide.

In the embodiment shown in Figures 4 to 6, the retaining means 109 are preferably made of two through buckles 115, 116 since the buckles easily allow sliding and then adjusting the length of the elastic strips and of a seam to fasten the strip 117.

Preferably, the system 1 to protect the posture comprises three elastic strips 105, 106 and 107 connected to the coupling system 108.

Only the simultaneous presence of the three elastic strips 105, 106 and 107, in fact, allows keeping the user in the correct posture both during dynamic and static activities.

The elastic strips 105, 106 and 107 comprise respective ends 105a, 105b, 106a, 106b, 107a, 107b and central portions 105c, 106c, 107c.

The elastic strip 107 can be made of two distinct strip portions, both connected to their respective ends and irremovably to the coupling system 108.

More in detail, and as shown in detail in Figures 4 to 6, the vest 101a comprises:
- a first fastening portion 118a1 arranged on the descending part of the trapezius muscle between the base of the neck and the humerus, approximately above the left shoulder,
- a second fastening portion 118b1 arranged on the descending part of the trapezius muscle between the base of the neck and the humerus, approximately above the right shoulder,
- a third fastening portion 118a2 arranged at the height of the left hypochondrium,
- a fourth fastening portion 18b2 arranged at the height of the right hypochondrium,
- a fifth fastening portion 119a arranged on the left side,
- a sixth fastening portion 119b arranged on the right side.

The first end 105a of the first elastic strip 5 is fastened in an immovable way, preferably by means of a seam, to the fourth fastening portion 118a2.

The central portion 105c of the first strip engages the retaining means 119 of the coupling system 108 and in particular engages the buckle 115. The second end 105b of the first strip 105 is removably fastened to the second fastening portion 119b, preferably by means of a Velcro system.

A first end 106a of the second elastic strip 106 is irremovably fastened, preferably by means of a seam, to the third fastening portion 118a2. The central portion 106c of the second strip engages the retaining means 109 of the coupling system 108 and in particular a buckle 116. The second end 106b is fastened to the fifth fastening portion 119a.

A first end 107a of the third elastic strip 107 is fastened to the first fastening portion 118a1.

The central portion 107c of the third strip engages the retaining means 109 of the coupling system 108 and in particular the notch 117.

Alternatively, the central portion 107c may also not be there and the strip 107 may be an integral part of the coupling system 108.

The second end 107b is irremovably fastened, preferably by means of a seam, to the second fastening portion 118b1.

The strip 107 made of two portions 107f, 107 is therefore irremovably fastened to both its ends and cannot slide and be adjusted.

The tension of the elastic strips 105, 106 by positioning the elastic strips on the respective fastening portions 119a, 119b is adjusted to allow correcting the tension and therefore the user's posture.

In detail, when the user wears the vest or shirt, she/he only fastens the elastic strips 105b and 106b on the respective fastening portions and, by adjusting the tension, adjusts the posture correction.

In fact, the more stretched the elastic strips, the greater the posture correction effect.

With respect to the first embodiment described above, this second embodiment allows a simplified adjustment and a simplified overall structure of the garment.

Preferably, the elastic strips 105, 106 are releasably fastened to the respective fastening portions, more preferably by means of a hook and loop system, even more preferably with a hook and loop system known as Velcro®.

Vice versa, the elastic strip 107 is irremovably fastened, preferably by means of a seam.

The posture correction system 1, 103 allows an effective correction only in combination with the t-shirt or vest.

Advantageously, the first fastening portion 118a1 and the second fastening portion 118a2 can be connected by a reinforcing area of the t-shirt 130, which extends from the left shoulder to the left hypochondrium.

Advantageously, the third fastening portion 118b1 and the fourth fastening portion 118b2 can be connected by a reinforcing area of the shirt 131, which extends from the right shoulder to the right hypochondrium.

Advantageously, the fifth fastening portion 119a and the sixth fastening portion 119b can form a single fastening portion along the whole lumbar back portion.

The elastic strips 5, 6, 7, 105, 106 and 107 have a suitable width to have an effective posture correction effect and which is preferably comprised between 3 and 10 cm.

The width of the elastic strips 5, 6, 7, 105, 106 and 107 can be variable along their length.

The elastic strips 5, 6, 7, 105, 106 and 107 have a suitable length to be fastened in different positions on the relative fastening portions to ensure an adjustment of the position and therefore a greater or lesser posture correction effect and so that the garment is tailored on the user's size.

The strips 5, 6, 7, 105, 106 and 107 therefore preferably have a length comprised between 25 cm and 60 cm.

Optionally and preferably, it is possible that the garment 1 comprises further elastic strips.

Advantageously, the garment 1 could optionally comprise a fourth elastic strip 20 arranged around the lumbar strip and in particular connecting the portions 19a and 19b.

Preferably, the elastic strips 5 and 6 are fastened to the respective fastening portions at more convenient angles to ensure a maximum posture correction.

More preferably, the elastic strip 107 is fastened at an angle comprised between 0° and 30° with respect to the vertical axis A, more preferably between 0° and 20°.

More preferably, the elastic strip 105 has the upper end 105b fastened at an angle comprised between 75° and 105° with respect to the vertical axis A.

More preferably, the elastic strip 105 has the lower end 105a fastened at an angle comprised between 120° and 150° with respect to the vertical axis A.

More preferably, the elastic strip 106 has the upper end 106b fastened at an angle comprised between 75° and 105° with respect to the vertical axis A.

More preferably, the elastic strip 106 has the lower end 106a fastened at an angle comprised between 120° and 150° with respect to the vertical axis A.

Preferably, the t-shirt 101a may be provided with a front hinge, in a preferably central position with respect to the user's chest, which allows its being worn by users who have a limited ability to move.

Preferably, the elastic strips 5, 6, 7, 105, 106, 107 and 20 are made of a material provided with a specific and predetermined elasticity and which can be more or less elongated or stretched during their fastening.

The intrinsic elasticity of the elastic strips 5, 6, 7, 105, 106, 107 and optionally 20 is essential in ensuring a correct posture correction while avoiding any hindrance to the user's movements.

Preferably, the elastic strips, 6, 7, 105, 106, 107 and 20 are made of one or more materials having a total elongation comprised between 100 and 250% when subjected to a force of 400 N, more preferably between 150 and 200%.

Preferably, the elastic strips comprise a multilayer material. Preferably, the multilayer material comprises at least one layer of breathable elastic fabric and a layer comprising thermoadhesive polyurethane.

More preferably, the multilayer material comprises four base layers.

Preferably, the multilayer material is made of a fabric composed of a layer of breathable elastic fabric and a layer of polyurethane for a total of 285 (+/- 15) gr/m² and a second layer with the same characteristics. The total fabric obtained is a multilayer fabric of 570 (+/- 15) gr/m².

Laboratory tests have been carried out to verify the elongation of an elastic strip according to the present invention, when the elastic strip is formed with the most preferred materials listed above. It has been verified that the elongation of the elastic strip is 170% at 400N and is 160% at 400N if only the multilayer material composed of 4 layers is considered.

Advantageously, the t-shirt or vest 1 may extend in length to the buttocks to facilitate the anchorage of the shirt to the buttocks.

The advantages of the garment of the present invention are clear. In particular, it allows ensuring a correct posture both while the user is static and when she/he is engaged in a dynamic activity.

Moreover, thanks to the fact that the elastic strips 5, 6 and 7 can be fastened on a large portion of the fastening portions 18a1, 18a2, 18b1, 18b2, 19a, 19b, the tension and the garment may be better adjusted to have a more or less accentuated posture correction and to better suit the size of the user.

Vice versa, when the elastic strips 105, 16, 107 are at least partially irremovably fastened.

Furthermore, the garment of the present invention does not include portions of metallic, plastic, composite and ceramic materials.

It is also easily wearable despite its complexity and is easily stored and invisible under other garments.

Moreover, the garment of the present invention is light and breathable and wearable as an undergarment even during sports activities.

Since the garment is almost invisible, the user is more encouraged to wear it and this ensures a posture improvement.

## Claims

1. Garment (1, 101) including a t-shirt (1a, 101a) and a system (103) to correct the posture, comprising:
- a first elastic strip (5, 105), a second elastic strip (6,106) and a third elastic strip (7, 107), each elastic strip comprising a first end (5a, 6a, 7a, 105a, 106a, 107a) and a second end (5b, 6b, 7b, 105b, 106b, 107b),
- a coupling system (8, 108) arranged on the back (1a2, 101a2) of the t-shirt and being provided with a plurality of retaining means (15, 16, 115, 116) for the elastic strips (5, 6, 7, 105, 106, 107),
- a first fastening portion (18al, 118a1) arranged on the t-shirt in an area corresponding to the upper portion of the torso between the left shoulder and the neck or in an area corresponding to the descending part of the trapezius muscle between the base of the neck and the humerus above the left shoulder,
- a second fastening portion (18bl, 118b1) arranged on the t-shirt in an area corresponding to the upper portion of the torso between the right shoulder and the neck or in an area corresponding to the descending part of the trapezius muscle between the base of the neck and the humerus approximately above the right shoulder,
- a third fastening portion (18a2, 118a2) arranged on the t-shirt in an area corresponding to the left hypochondrium,
- a fourth fastening portion (18b2, 118b2) arranged on the t-shirt in an area corresponding to the right hypochondrium,
- a fifth fastening portion (19a, 119a) arranged on the left side of the t-shirt,
- a sixth fastening portion (19b, 119b) arranged on the right side of the t-shirt, wherein
- a first end (5a, 105a) of said first elastic strip (5, 105) is fastened to said fourth fastening portion (18b2, 118b2),
- a central portion (5c, 105c) of said first elastic strip (5, 105) engages retaining means (109, 109) of said coupling system (8, 108),
- a second end (5b, 105b) of said first elastic strip is fastened to said sixth fastening portion (19b, 119b),
- a first end (6a, 106a) of said second elastic strip (6, 106) is fastened to said third fastening portion (18a2, 118a2),
- a central portion (6c, 106c) of said second elastic strip (6, 106) engages said retaining means (9, 109) of said coupling system (8, 108),
- a second end (6b, 106b) of said second elastic strip (6, 106) is fastened to said fifth fastening portion (19a),
- a first end (7a, 107a) of said third elastic strip (7, 107) is fastened to said first fastening portion (18al, 118a1),
- a central portion (7c, 107c) of said third elastic strip (7, 107) engages said retaining means (9, 109) of said coupling system (8, 108),
- a second end (7b, 107b) of said third strip (7, 107) fastened to said second fastening portion (18b1, 118b1) wherein said elastic strips (5, 6, 7, 105, 106, 107) are fastened in a releasable manner and in that said elastic strips (5, 6, 7, 105, 106, 107) have an elongation comprised between 150% and 200% when subjected to a force of 400 N.

2. A garment (1) according to claim 1, **characterised in that** it comprises a fourth elastic strip (20) .

3. A garment (1) according to claims 1 or 2, **characterised in that** said fourth elastic strip (20) is fastened at a first end (20a) to said fifth fastening portion (19a) and is fastened at a second end (20b) to said sixth fastening portion (19b).

4. A garment (1) according to any one the preceding claims, **characterised in that** said elastic strips (5, 6, 7, 105, 106, 107) are fastened with a hook and loop or Velcro® system.

5. A garment (1) according to any one of claims 1 to 3, **characterised in that** at least one of said elastic strips (5, 6, 7, 105, 106, 107) is fastened in a non-releasable manner.

6. A garment (1) according to claim 5, **characterised in that** at least two of said elastic strips (5, 6, 7, 105, 106, 107) are fastened at least at one end in a non-releasable manner.

7. A garment (1) according to any one of claims 5 to 6, **characterised in that** a first end (7a, 107a) of said third elastic strip (7, 107) is fastened to said first fastening portion (18al, 118a1) in a non-releasable manner, preferably by sewing, and **in that** a second end of said third elastic strip (7, 107) is fastened to said second fastening portion (118b1) in a non-releasable manner, preferably by sewing.

8. A garment (1) according to any one the preceding claims, **characterised in that** a first end (105a) of said first elastic strip (105) is fastened to said fourth fastening portion (118b2) in a non-releasable manner, preferably by sewing, and **in that** a first end (106a) of said second elastic strip (106) is fastened to said third fastening portion (118a2) in a non-releasable manner, preferably by sewing.

9. A garment (1) according to any one the preceding claims, **characterised in that** said elastic strips (5, 6, 7, 105, 106, 107) have a length comprised between 25 cm and 60 cm.

10. A garment (1) according to any one the preceding claims, **characterised in that** said elastic strips (5, 6, 7) have a width comprised between 3 cm and 10 cm.

## Patentansprüche

1. Kleidungsstück (1, 101) mit einem T-Shirt (1a, 101a) und einem System (103) zur Korrektur der Körperhaltung, das Folgendes aufweist:
- ein erstes elastisches Band (5, 105), ein zweites elastisches Band (6, 106) und ein drittes elastisches Band (7, 107), wobei jedes Band ein erste Ende (5a, 6a, 7a, 105a, 106a, 107a) und ein zweites Ende (5b, 6b, 7b, 105b, 106b, 107b) aufweist,
- ein Kopplungssystem (8, 108), das auf der Rückseite (1a2, 101a2) des T-Shirts angeordnet ist und mit mehreren Haltevorrichtungen (15, 16, 115, 116) für die elastischen Bänder (5, 6, 7, 105, 106, 107) versehen ist,
- einen ersten Befestigungsabschnitt (18a1, 118a1), der auf dem T-Shirt in einem Bereich angeordnet ist, der dem oberen Abschnitt des Oberkörpers zwischen der linken Schulter und dem Hals entspricht, oder in einem Bereich angeordnet ist, der dem absteigenden Teil des Trapezmuskels zwischen dem Halsansatz und dem Humerus oberhalb der linken Schulter entspricht,
- einen zweiten Befestigungsabschnitt (18b1, 118b1), der auf dem T-Shirt in einem Bereich angeordnet ist, der dem oberen Abschnitt des Oberkörpers zwischen der rechten Schulter und dem Hals entspricht, oder in einem Bereich angeordnet ist, der dem absteigenden Teil des Trapezmuskels zwischen dem Halsansatz und dem Humerus ungefähr oberhalb der rechten Schulter entspricht,
- einen dritten Befestigungsabschnitt (18a2, 118a2), der auf dem T-Shirt in einem Bereich angeordnet ist, der dem linken Hypochondrium entspricht,
- einen vierten Befestigungsabschnitt (18b2, 118b2), der auf dem T-Shirt in einem Bereich angeordnet ist, der dem rechten Hypochondrium entspricht,
- einen fünften Befestigungsabschnitt (19a, 119a), der auf der linken Seite des T-Shirts angeordnet ist,
- einen sechsten Befestigungsabschnitt (19b, 119b), der auf der rechten Seite des T-Shirts angeordnet ist, wobei
- ein erstes Ende (5a, 105a) des ersten elastischen Bands (5, 105) am vierten Befestigungsabschnitt (18b2, 118b2) befestigt ist,
- ein Mittelabschnitt (5c, 105c) des ersten elastischen Bands (5, 105) in die Haltevorrichtung (109, 109) des Kopplungssystems (8, 108) eingreift,
- ein zweites Ende (5b, 105b) des ersten elastischen Bands am sechsten Befestigungsabschnitt (19, 119b) befestigt ist,
- ein erstes Ende (6a, 106a) des zweiten elastischen Bands (6, 106) am dritten Befestigungsabschnitt (18a2, 118a2) befestigt ist,
- ein Mittelabschnitt (6c, 106c) des zweiten elastischen Bands (6, 106) in die Haltevorrichtung (9, 109) des Kopplungssystems (8, 108) eingreift,
- ein zweites Ende (6b, 106b) des zweiten elastischen Bands (6, 106) am fünften Befestigungsabschnitt (19a) befestigt ist,
- ein erstes Ende (7a, 107a) des dritten elastischen Bands (7, 107) am ersten Befestigungsabschnitt (18a1, 118a1) befestigt ist,
- ein Mittelabschnitt (7c, 107c) des dritten elastischen Bands (7, 107) in die Haltevorrichtung (9, 109) des Kopplungssystems (8, 108) eingreift,
- ein zweites Ende (7b, 107b) des dritten Bands (7, 107) am zweiten Befestigungsabschnitt (18b1, 118b1) befestigt ist,
- wobei die elastischen Bänder (5, 6, 7, 105, 106, 107) lösbar befestigt sind und die elastischen Bänder (5, 6, 7, 105, 106, 107) eine Längenausdehnung zwischen 150 % und 200 % aufweisen, wenn sie einer Kraft von 400 N ausgesetzt sind.

2. Kleidungsstück (1) nach Anspruch 1, **dadurch gekennzeichnet, dass** es ein viertes elastisches Band (20) aufweist.

3. Kleidungsstück (1) nach Anspruch 1 oder 2, **dadurch gekennzeichnet, dass** das vierte elastische Band (20) mit einem ersten Ende (20a) am fünften Befestigungsabschnitt (19a) befestigt ist und mit einem zweite Ende (20b) am sechsten Befestigungsabschnitt (19b) befestigt ist.

4. Kleidungsstück (1) nach einem der vorstehenden Ansprüche, **dadurch gekennzeichnet, dass** die elastischen Bänder (5, 6, 7, 105, 106, 107) mit einem Klettverschluss oder Velcro®-System befestigt sind.

5. Kleidungsstück (1) nach einem der Ansprüche 1 bis 3, **dadurch gekennzeichnet, dass** mindestens eines der elastischen Bänder (5, 6, 7, 105, 106, 107) nicht lösbar befestigt ist.

6. Kleidungsstück (1) nach Anspruch 5, **dadurch gekennzeichnet, dass** mindestens zwei der elastischen Bänder (5, 6, 7, 105, 106, 107) an mindestens einem Ende nicht lösbar befestigt sind.

7. Kleidungsstück (1) nach einem der Ansprüche 5 bis 6, **dadurch gekennzeichnet, dass** ein erstes Ende (7a, 107a) des dritten elastischen Bands (7, 107) nicht lösbar am ersten Befestigungsabschnitt (18a1, 118a1) befestigt ist, vorzugsweise durch Nähen, und dass ein zweites Ende des dritten elastischen Bands (7, 107) nicht lösbar am zweiten Befestigungsabschnitt (118b1) befestigt ist, vorzugsweise durch Nähen.

8. Kleidungsstück (1) nach einem der vorstehenden Ansprüche, **dadurch gekennzeichnet, dass** ein erstes Ende (105a) des ersten elastischen Bands (105) nicht lösbar am vierten Befestigungsabschnitt (118b2) befestigt ist, vorzugsweise durch Nähen, und dass ein erstes Ende (106a) des zweiten elastischen Bands (106) nicht lösbar am dritten Befestigungsabschnitt (118a2) befestigt ist, vorzugswiese durch Nähen.

9. Kleidungsstück (1) nach einem der vorstehenden Ansprüche, **dadurch gekennzeichnet, dass** die elastischen Bänder (5, 6, 7, 105, 106, 107) eine Länge zwischen 25 cm und 60 cm aufweisen.

10. Kleidungsstück (1) nach einem der vorstehenden Ansprüche, **dadurch gekennzeichnet, dass** die elastischen Bänder (5, 6, 7) eine Breite zwischen 3 cm und 10 cm aufweisen.

## Revendications

1. Vêtement (1, 101) comprenant un T-shirt (1a, 101a) et un système (103) pour corriger la posture, comprenant :
- une première bande élastique (5, 105), une deuxième bande élastique (6, 106) et une troisième bande élastique (7, 107), chaque bande élastique comprenant une première extrémité (5a, 6a, 7a, 105a, 106a, 107a) et une deuxième extrémité (5b, 6b, 7b, 105b, 106b, 107b),
- un système d'assemblage (8, 108) disposé sur le dos (1a2, 101a2) du T-shirt et pourvu d'une pluralité de moyens de retenue (15, 16, 115, 116) pour les bandes élastiques (5, 6, 7, 105, 106, 107),
- une première partie de fixation (18a1, 118a1) disposée sur le T-shirt dans une zone correspondant à la partie supérieure du torse entre l'épaule gauche et le cou ou dans une zone correspondant à la partie descendante du muscle trapèze entre la base du cou et l'humérus au-dessus de l'épaule gauche,
- une deuxième partie de fixation (18b1, 118b1) disposée sur le T-shirt dans une zone correspondant à la partie supérieure du torse entre l'épaule droite et le cou ou dans une zone correspondant à la partie descendante du muscle trapèze entre la base du cou et l'humérus à peu près au-dessus de l'épaule droite,
- une troisième partie de fixation (18a2, 118a2) disposée sur le T-shirt dans une zone correspondant à l'hypocondre gauche,
- une quatrième partie de fixation (18b2, 118b2) disposée sur le T-shirt dans une zone correspondant à l'hypocondre droit,
- une cinquième partie de fixation (19a, 119a) disposée sur le côté gauche du T-shirt,
- une sixième partie de fixation (19b, 119b) disposée sur le côté droit du T-shirt, dans lequel
- une première extrémité (5a, 105a) de ladite première bande élastique (5, 105) est fixée à ladite quatrième partie de fixation (18b2, 118b2),
- une partie centrale (5c, 105c) de ladite première bande élastique (5, 105) est en prise avec des moyens de retenue (109, 109) dudit système d'assemblage (8, 108),
- une deuxième extrémité (5b, 105b) de ladite première bande élastique est fixée à ladite sixième partie de fixation (19b, 119b),
- une première extrémité (6a, 106a) de ladite deuxième bande élastique (6, 106) est fixée à ladite troisième partie de fixation (18a2, 118a2),
- une partie centrale (6c, 106c) de ladite deuxième bande élastique (6, 106) est en prise avec lesdits moyens de retenue (9, 109) dudit système d'assemblage (8, 108),
- une deuxième extrémité (6b, 106b) de ladite deuxième bande élastique (6, 106) est fixée à ladite cinquième partie de fixation (19a),
- une première extrémité (7a, 107a) de ladite troisième bande élastique (7, 107) est fixée à ladite première partie de fixation (18a1, 118a1),
- une partie centrale (7c, 107c) de ladite troisième bande élastique (7, 107) est en prise avec lesdits moyens de retenue (9, 109) dudit système d'assemblage (8, 108),
- une deuxième extrémité (7b, 107b) de ladite troisième bande (7, 107) est fixée à ladite deuxième partie de fixation (18b1, 118b1),
dans lequel lesdites bandes élastiques (5, 6, 7, 105, 106, 107) sont fixées d'une manière détachable et en ce que lesdites bandes élastiques (5, 6, 7, 105, 106, 107) ont un allongement compris entre 150 % et 200 % lorsqu'elles sont soumises à une force de 400 N.

2. Vêtement (1) selon la revendication 1, **caractérisé en ce qu'**il comprend une quatrième bande élastique (20).

3. Vêtement (1) selon la revendication 1 ou 2, **caractérisé en ce que** ladite quatrième bande élastique (20) est fixée à une première extrémité (20a) à ladite cinquième partie de fixation (19a) et est fixée à une deuxième extrémité (20b) à ladite sixième partie de fixation (19b).

4. Vêtement (1) selon l'une quelconque des revendications précédentes, **caractérisé en ce que** lesdites bandes élastiques (5, 6, 7, 105, 106, 107) sont fixées avec un crochet et une boucle ou un système Velcro®.

5. Vêtement (1) selon l'une quelconque des revendications 1 à 3, **caractérisé en ce qu'**au moins une desdites bandes élastiques (5, 6, 7, 105, 106, 107) est fixée d'une manière non détachable.

6. Vêtement (1) selon la revendication 5, **caractérisé en ce qu'**au moins deux desdites bandes élastiques (5, 6, 7, 105, 106, 107) sont fixées au moins à une extrémité d'une manière non détachable.

7. Vêtement (1) selon l'une quelconque des revendications 5 à 6, **caractérisé en ce qu'**une première extrémité (7a, 107a) de ladite troisième bande élastique (7, 107) est fixée à ladite première partie de fixation (18a1, 118a1) d'une manière non détachable, de préférence par couture, et **en ce qu'**une deuxième extrémité de ladite troisième bande élastique (7, 107) est fixée à ladite deuxième partie de fixation (118b1) d'une manière non détachable, de préférence par couture.

8. Vêtement (1) selon l'une quelconque des revendications précédentes, **caractérisé en ce qu'**une première extrémité (105a) de ladite première bande élastique (105) est fixée à ladite quatrième partie de fixation (118b2) d'une manière non détachable, de préférence par couture, et **en ce qu'**une première extrémité (106a) de ladite deuxième bande élastique (106) est fixée à ladite troisième partie de fixation (118a2) d'une manière non détachable, de préférence par couture.

9. Vêtement (1) selon l'une quelconque des revendications précédentes, **caractérisé en ce que** lesdites bandes élastiques (5, 6, 7, 105, 106, 107) ont une longueur comprise entre 25 cm et 60 cm.

10. Vêtement (1) selon l'une quelconque des revendications précédentes, **caractérisé en ce que** lesdites bandes élastiques (5, 6, 7) ont une largeur comprise entre 3 cm et 10 cm.
